# EUROPEAN PATENT APPLICATION

(11) **EP 1 797 934 A1**
(43) Date of publication of application: **20.06.2007**
(21) Application number: 05781937.7
(22) Date of filing: 06.09.2005
(51) Int. Cl.: B01D 9/02, A61K 9/16

(54) **PROCESS FOR PRODUCING FINELY PARTICULATE SUBSTANCE AND FINELY PARTICULATE SUBSTANCE**

(30) Priority: 07.09.2004 JP 2004259487
(71) Applicant: Mitsubishi Chemical Corporation, Minato-ku Tokyo 108-0014 (JP)
(72) Inventor: SAITA, S., MITSUBISHI CHEMICAL GROUP SCIENCE AND, Yokohama-shi, Kanagawa, 2278502 (JP); SEKI, H., MITSUBISHI CHEMICAL GROUP SCIENCE AND, Yokohama-shi, Kanagawa, 2278502 (JP); ASATANI, H., MITSUBISHI CHEMICAL GROUP SCIENCE AND, Yokohama-shi, Kanagawa, 2278502 (JP)
(74) Representative: HOFFMANN EITLE
(86) International application number: PCT/JP2005/016301
(87) International publication number: WO 2006/028074

(57) **Abstract**

A method of producing fine particle-like materials formed by a crystallization method, which producing method is capable of producing the fine particles with a narrow particle size distribution and also capable of inhibiting aggregation of the fine particles without using any dispersant; and the fine particle-like materials, are provided. The present method of producing the fine particles by crystallization comprises preparing a solution containing the material to be finely divided, and bringing this solution into contact with a substrate having the microprojections provided on its surface at a density of not less than 100 projections/cm² to cause precipitation of the fine particles. The fine particles produced by the above method are those of physiological active materials containing no dispersant.

## Description

### TECHNICAL FIELD

The present invention relates to a method of producing fine particle-like materials, and to the fine particle-like materials as well.

### BACKGROUND ART

Hitherto, many attempts have been made for the application of fine particles in a variety of chemical products such as luminous elements and diagnostic medicaments using semiconductor substances, ultra high-density recording media using magnetic materials, catalysts using metallic materials, and physiological active materials such as pharmaceuticals using organic compounds. For instance, the fine particles of the organic compounds only slightly soluble in water with a solubility of less than 10 mg/ml are applied in a wide variety of chemical products such as pharmaceuticals, ink, dyes, pigments, lubricants, insecticides, agricultural chemicals, fertilizers and cosmetics. In application of such fine particles, for instance, in pharmaceuticals, especially medicines only sparingly soluble in water, there are cases where the medicinal material is very slow to elute and not sufficiently eluted even after it has passed its absorbing region in the body. In order to solve such a problem, there has been proposed a technique for elevating the bioavailability by finely dividing the material to a nanosize level of particles to increase the specific surface area and thereby improve the dissolving rate of the material.

Many proposals have been made on the method for producing the nanosize particles. There are roughly two types of method now available for the said purpose:
breakdown method in which the bulk is pulverized to a nanosize level, and build-up method in which the cluster size particles are grown up to a nanosize. Included in the category of breakdown method is, for instance, a ball mill method (see, for example, Patent Document 1). This method is capable of forming the pharmaceutical particles smaller than 400 nm, but it has such a disadvantage that mixing of foreign matters such as grinder material is unavoidable. On the other hand, as a technique that can be classified in the category of build-up method which is free of the problem of the said contamination, there can be cited a technique making use of crystallization in which the fine particles of solute are precipitated from a supersaturated solution of the solute (see, for instance, Patent Document 2). It is possible with this method to obtain the fine pharmaceutical particles with a size of, for example, as small as 156 nm, but since this method is greatly affected by the type of the solute used and its compositional ratio in the pharmaceuticals, it has such a drawback that the setting of the optimal conditions is difficult.

Also, both of the breakdown and build-up methods have such disadvantages that control of the obtained particle size is difficult and also the particle size distribution becomes broad. Further, since the nanosize particles have a tendency to aggregate with each other, it is necessary in both methods to use a dispersant such as a surfactant for preventing aggregation of the particles, which makes mixing of a compound(s) alien to the pharmaceuticals unavoidable.
Patent Document 1: USP 5,145,684
Patent Document 2: US Pat. Appln. Laid-Open No. 2003/0049323

### DISCLOSURE OF THE INVENTION

### PROBLEM TO BE SOLVED BY THE INVENTION

The present invention has been attained in view of the above-described prior art problems, and its object is to provide a method of producing fine particle-like materials by a crystallization method, by which the method of producing fine particle-like materials, fine particles having a narrow particle size distribution can be obtained and aggregation of the particles with each other can be prevented without using any dispersant. The present invention is also envisaged to provide such fine particle-like materials.

### MEANS FOR SOLVING THE PROBLEM

As a result of the present inventors' earnest studies for solving the above problems, it has been found that it was possible to produce the fine particles with a narrow particle size distribution and containing no dispersant without causing aggregation of the particles, by preparing a supersaturated solution of the material and bringing it into contact with a substrate having a plurality of microprojections on its surface in carrying out a crystallization method. The present invention was attained on the basis of the above findings.

In a first aspect of the present invention, there is provided a method of producing fine particle-like materials formed by a crystallization method, which comprises preparing a solution containing a material to be finely divided, and bringing this solution into contact with a substrate having microprojections provided on the surface thereof at a density of not less than 100 projections/cm² to precipitate fine particles.

In a second aspect of the present invention, there are provided the fine particles of physiological active materials obtained by the above-described method, the said particles containing no dispersant.

### EFFECT OF THE INVENTION

According to the present invention, it is possible to obtain the fine particles with a narrow particle size distribution and to inhibit aggregation of the particles without using a dispersant. Therefore, in the case of, for example, a pharmaceutical material which is only slightly soluble in water, it is possible to enhance bioavailability by increasing the specific surface area and elevating the dissolving rate by fine particle-like material. It is also possible to adjust timing of intake into the body by uniformizing the particle size distribution. Further, the fine particle-like materials according to the present invention are expected to find various applications as the physiological active substances such as nanosized particulate pharmaceuticals containing no undesirable compounds such as dispersant.

### BEST MODE FOR CARRYING OUT THE INVENTION

The materials to be finely divided by the crystallization method (base materials) in the present invention are not subject to any specific restrictions as far as they are soluble in solvents. The solubility of the base materials for solvents is usually not less than 1 mg/ml, preferably not less than 5 mg/ml. Particularly favorable for the treatment in the present invention are the component materials of pharmaceuticals, ink, pigments, cosmetics and the like which are preferably composed of the fine particles with an average size of less than 1 µm. Especially, in the case of the pharmaceuticals only slightly soluble in water (with their solubility in 20°C water being usually not more than 100 mg/ml, preferably not more than 10 mg/ml), it is possible to enhance their vital utility factor by finely dividing the base material to increase its specific surface area and to thereby elevate the dissolving rate. Further, since the timing of intake into the body can be adjusted by uniformizing the particle size distribution, administration of the pharmaceuticals in a pharmaceutically most appropriate way is made possible.

The solvent can be properly selected from those having a base material solubility of usually not less than 1 mg/ml, preferably not less than 5 mg/ml, according to the type of the base material. The solvent is preferably one which is liquid at a temperature of at least 0 to 30°C, particularly at 20 to 30°C. Examples of the solvents usable in the present invention include water; polar solvents such as alcohols, acetone, tetrahydrofuran (THF), methyl ethyl ketone (MEK) and dimethyl sulfoxide (DMSO); and non-polar solvents such as ethers, toluene and chloroform.

In case where the base material is soluble in water, water is preferably used as solvent, and when the base material is an oil-soluble compound, use of a non-polar solvent is preferred. Considering the change of solubility by rise or drop of the solution temperature, a non-polar solvent may be used even in the case of a water-soluble base material. The opposite combinations are also possible.

In the present invention, the substrate with which a solution containing a base material in a supersaturated state is brought into contact is a plate provided with a plurality of microprojections on the surface thereof. It is essential that such microprojections be provided at a density of not less than 100 projections/cm²; preferably they are provided at a density of not less than 10,000 projections/cm², more preferably not less than 100,000,000 projections/cm². The upper limit of density of microprojections is usually 10,000,000,000 projections/cm². If the density of microprojections is below the above-defined range, it is difficult to produce the objective fine particles.

The microprojections may take various configurations such as conical, truncated-conical, polygonal pyramidal, polygonal truncated-pyramidal, columnar, and polygonal prismatic. The configuration of microprojections is not defined, but in view of the probability that such microprojection configuration may affect the form of the precipitated fine particles, it is preferable that the individual microprojections provided at the said density be substantially uniform in configuration for producing the fine particles with a narrow particle size distribution. Substantially identical configuration of the whole microprojections is also preferable in view of, for instance, facilitation in making the substrate having the microprojections. The lower limit of height of the microprojections is usually 10 nm, preferably 50 nm, and the upper limit is usually 5,000 nm, preferably 1,000 nm.

It is also preferable that the arrangement of the microprojections on the substrate surface would have certain regularity in terms of particle size distribution. For instance, they may be arrayed in zigzag arrangement, hexagonal packed arrangement or cubic packed arrangement.

The material of the substrate having microprojections on the surface is not specifically restricted as far as it enables formation of the said microprojections, is resistant to the solvent of the solution with which the substrate is to be contacted, and also does not cause any chemical reaction with the solute. Preferably, the substrate material is of the type which has no likelihood of giving rise to physical phenomena such as adsorption. Typical examples of such material are metals such as iron, nickel and aluminum, their alloys, glass and plastic.

For forming the microprojections, there are available, for example, a method in which nickel is electroformed on a basal plate which has been patterned by semiconductor lithography or interference exposure, and a method in which vapor-phase growth of a semiconductor is conducted on a basal plate to induce self organization of insular projections. The method utilizing lithography is preferred in view of ease of control of projection configuration. Since the degree of precipitation of fine particles is variable depending on the chemical properties of the projection surface, the projection surface may have been subjected to a hydrophobic or hydrophilic treatment.

In the method of producing the fine particle-like materials according to the present invention, a solution containing a base material in a supersaturated state is prepared, and this solution is brought into contact with the said substrate having the said microprojections on the surface. In this process, a solution with lower than saturation solubility of the base material may be rendered into a supersaturated state and then brought into contact with the said substrate. However, in order to let the fine particles of the base material precipitate regularly at the tops and/or the sides and roots of the microprojections on the substrate surface, it is more preferable that the solution with lower than saturation solubility of the base material be initially brought into contact with the said substrate and then turned into a supersaturated state.

For rendering the solution with lower than saturation solubility into a supersaturated state, there are available, for example, a method in which the temperature of the solution is lower and a method in which the solvent is evaporated to lower concentration of the solute, but the former method is preferred for ease of operation. For lowering the solution temperature, it is preferable to lower temperature of the substrate so as to effectuate corresponding lowering of solution temperature through heat transfer. Temperature is lowered within the range of usually 1 to 100°C.

In the present invention, as a result of contact of the solution containing a base material in a supersaturated state with the said substrate, the fine particles of the base material are deposited at the peripheral parts, such as tops and/or sides and roots of the microprojections on the substrate surface. The precipitated particles of the base material may be either crystalline or non-crystalline. In the case of the crystalline particles, it is possible to control the crystal system of the precipitated particles by crystal structure of the surfaces of the microprojections on the substrate surface.

In the present invention, the fine particles precipitated in the manner described above are recovered after removing the residual solution. When the conventional method is used for producing the fine particles, an intricate operation such as filtration or centrifuging is required for separating the fine particles and the residual solution, but according to the process of the present invention, separation of the fine particles and the residual solution can be easily effected by, for instance, a method in which the whole substrate is washed with a poor solvent for the particles or a method in which the residual solution is blown away with an inert gas such as air or nitrogen, or a combination of these methods. Further, after removal of the residual solution, the fine particles can be recovered in the form of a slurry by various methods, such as conducting a supersonic treatment in a poor solvent, flowing a solvent with a medium degree of solubility, or heating the substrate immersed in a poor solvent.

The obtained slurry of fine particles stays deposited in the neighborhood of the microprojections on the substrate surface, thus preventing the particles from contacting each other, until the particles are separated from the substrate after deposition on the substrate surface, so that the particles are inhibited from aggregating with each other, and therefore by quickly carrying out the succeeding operations, it is possible to use the nanosize particles in a state free of aggregation. Even if such aggregation of the particles should have occurred to a certain degree, they can be easily redispersed by a simple method such as supersonic treatment.

The production method of the present invention excels in controllability of the size of fine particles and particle size distribution, and the average size (diameter) of the obtained fine particles can be controlled within the range of usually not less than 1 nm and less than 1 mm, preferably not less than 1 nm and less than 500 µm, more preferably not less than 1 nm and less than 50 µm, most preferably not less than 1 nm and less than 1 µm. In the present invention, "average particle diameter" means weight-average particle diameter. Weight-average particle diameter can be determined by a dynamic light-scattering method.

The fine particles obtained according to the method of the present invention has a narrow particle size distribution as described below. That is, in the weight-converted particle size distribution of the fine particles obtained according to the present invention, the ratio of the particle diameters (D₉₀) of 90 wt% of undersize, which are the diameters representing the particle portion of up to 90% of the overall weight as integrated from the smaller particle diameter side, to the particle diameters (D₅₀) of 50 wt% of undersize, which are the diameters representing the particle portion of up to 50% of the overall weight, D₉₀/D₅₀. is usually not more than 2, preferably not more than 1.8, more preferably not more than 1.5. This endorses the presence of few coarse-sized particles in the fine particles obtained according to the present invention. Also, the ratio of the diameters (D₅₀) of 50 wt% of underside, which are the diameters representing the particle portion of up to 50% of overall weight, to the diameters (D₁₀) of 10 wt% of underside, which are the diameters representing the particle portion of up to 10% of overall weight, D₅₀/D₁₀, is usually not more than 2, preferably not more than 1.8, more preferably not more than 1.5. This particle size distribution indicates the presence of few ultra-small diameter particles, too. The above particle size distribution can be determined by a dynamic light-scattering method.

### EXAMPLES

### Example 1:

At room temperature of 20°C, 62 mg of L-glutamic acid was weighed out and put into and dissolved in 10 ml of water in a 30 ml phial with threaded top to prepare a solution. Solubility of L-glutamic acid in water at 20°C was 7.2 mg/ml while concentration of the prepared L-glutamic acid solution was 6.2 mg/ml, indicating that the water solubility of this acid was lower than the saturation solubility.

Meanwhile, an SUS valve having a 19 x 23 mm plane surface at the bottom was secured upside down, and placed thereon was a 9 x 10 mm, 0.3 mm thick substrate having its front side patterned with the 450 nm high conical nickel microprojections arranged at intervals of 450 nm crosswise at a density of 500,000,000 projections/cm² by semiconductor lithography, with the back side of the substrate being a flat surface. Then 0.05 g of the previously prepared solution was dropped onto the said substrate by a micropipette at room temperature to form the liquid droplets, and a 0°C coolant was continuously flown to the valve, maintaining this situation for 16 minutes. Since the solution of L-glutamic acid at 0°C was 3.3 mg/ml, it could be surmised that the solution has passed the state of supersaturation to cause precipitation of the fine particles of L-glumatic acid.

20 ml of water of room temperature was placed in a 5 cm-diameter Petri dish, and the substrate, with its both sides gripped by pincettes, was passed twice in its entirety through water in the Petri dish to wash away the remaining saturated solution. Immediately thereafter, nitrogen was blown against the substrate by a nitrogen gun to effect drying, thereby obtaining a sample. Observing the sample surface magnified 22,000 times by a scanning electron microscope, it was seen that the nano-particles, 180 nm in diameter, of L-glutamic acid were deposited at the tops of part of the microprojections.

Another similarly prepared sample was put into 10 ml of water in a 30 ml phial with threaded top, to which supersonic waves were applied to let the nano-particles of L-glutamic acid part from the substrate, and the particle size distribution of the slurry containing the thus obtained L-glutamic acid nano-particles was determined using Malvern's dynamic light-scattering particle size distribution meter "HPPS", finding that these nano-particles had an average diameter of approximately 180 nm. It was thus confirmed that these particles were the fine particles with low D₉₀/D₅₀ and D₅₀/D₁₀ ratios and a narrow particle size distribution. Also, the obtained L-glumatic acid nano-particles were free of dispersants such as surfactant.

### Comparative Example 1:

5 ml of the same solution as used in Example 1 was put into a 10 ml vial and the vial was immersed in a 0°C coolant and kept therein for 16 minutes, consequently forming a cloudy slurry of fine particles. The particle size distribution of this slurry was determined in the same way as in Example 1, finding that the produced particles had an average diameter of 8 µm and a wide particle size distribution with D₉₀/D₅₀ ratio of 3.0 and D₅₀/D₁₀ ratio of 2.5.

## Claims

1. A method of producing fine particle-like materials formed by a crystallization method, which comprises preparing a solution containing a material to be finely divided, and bringing this solution into contact with a substrate having microprojections provided on the surface thereof at a density of not less than 100 projections/cm² to precipitate fine particles.

2. The method according to Claim 1, wherein a solution containing the material to be finely divided in a non-saturated state is prepared, and this solution is brought into contact with the substrate having the microprojections provided on the surface thereof at a density of not less than 100 projections/cm² and then is rendered into a supersaturated state to cause precipitation of the particles.

3. The method according to Claim 1 or 2, wherein the fine particles have an average diameter of not less than 1 nm but less than 1 mm.

4. The method according to any one of Claims 1 to 3, wherein the ratio of the particle diameters (D₉₀) of 90 wt% of undersize to the particle diameters (D₅₀) of 50 wt% of undersize of the precipitated fine particles, D₉₀/D₅₀, is not more than 2.

5. The method according to any one of Claims 1 to 4, wherein the ratio of the particle diameters (D₅₀) of 50 wt% of underside to the particle diameters (D₁₀) of 10 wt% of undersize of the precipitated fine particles, D₅₀/D₁₀, is not more than 2.

6. The method according to any one of Claims 1 to 5, wherein the material to be finely divided is a physiological active material.

7. Fine particles of a physiological active material produced by the method of Claim 6, said fine particles containing no dispersant.
